# EUROPEAN PATENT APPLICATION

(11) **EP 4 339 958 A1**
(43) Date of publication of application: **20.03.2024**
(21) Application number: 22807240.1
(22) Date of filing: 25.03.2022
(51) Int. Cl.: G16B 45/00, C12P 21/08

(54) **ANTIBODY PRODUCTION ASSISTANCE METHOD AND ANTIBODY PRODUCTION ASSISTANCE PROGRAM**

(30) Priority: 13.05.2021 JP 2021081517
(71) Applicant: Shimadzu Corporation, Nakagyo-ku, Kyoto-shi, Kyoto 604-8511 (JP)
(72) Inventor: MOTOYAMA, Kento, Kyoto-shi, Kyoto 604-8511 (JP); YAMADA, Kaori, Kyoto-shi, Kyoto 604-8511 (JP); KANAI, Masaki, Kyoto-shi, Kyoto 604-8511 (JP); SUZUKI, Takashi, Kyoto-shi, Kyoto 604-8511 (JP)
(74) Representative: Kilian Kilian & Partner mbB
(86) International application number: PCT/JP2022/014461
(87) International publication number: WO 2022/239535

(57) **Abstract**

An assisting method for assisting production of an antibody includes: acquiring first time-series data corresponding to an amount of metabolite generated as a result of culturing in a small scale a cell line for producing the antibody under a predetermined culture condition (Step S12); acquiring second time-series data corresponding to an amount of metabolite generated as a result of culturing in a large scale the cell line under the predetermined culture condition (Step S12); specifying a difference between the first time-series data and the second time-series data (Step S14); specifying a target metabolite derived from the difference (Step S16); and generating display data based on the target metabolite (Step S17).

## Description

### TECHNICAL FIELD

The present disclosure relates to an antibody production assisting method and an antibody production assisting program.

### BACKGROUND ART

In recent years, studies in research on antibodies produced by culturing cells have been advanced. The specification of U.S. Patent No. 8911964 (PTL 1) discloses a technique related to production of antibodies by using culture of Chinese hamster ovary (CHO) cells. PTL 1 discloses that glucose concentrations is monitored and the monitored results is fed back.

### CITATION LIST

### PATENT LITERATURE

PTL 1: Specification of U.S. Patent No. 8911964

### SUMMARY OF INVENTION

### TECHNICAL PROBLEM

Incidentally, for production of antibodies, large-scaled mass production using large-size culture tanks are typically performed after manufacturing of antibodies in a laboratory level (small scale) using small-size culture tanks is tried.

According to this method, manufacturers specify culture conditions (for example, conditions under which antibodies can be appropriately manufactured) that are considered to be optimal in the small-scaled manufacturing of the antibodies. Also, the manufacturers manufacture the antibodies in a large scale under the same culture conditions as the culture conditions that are considered to be optimal. Theoretically, the antibodies should be appropriately manufactured even in the large scale by this method. However, there may be a case in which it is not possible to appropriately manufacture the antibodies in the large scale even if the antibodies are manufactured under the culture conditions that are considered to be optimal in the large scale due to differences in environments and the like between the small scale and the large scale. In addition, although it is preferable that the manufacturers specify appropriate culture conditions in the large scale, it is difficult to specify the culture conditions. In this case, a problem that it is difficult to produce the antibodies in the large scale may occur.

The present disclosure was made in order to solve such problems, and an object thereof is to assist production of antibodies in a large scale.

### SOLUTION TO PROBLEM

An antibody production assisting method according to a certain aspect of the present disclosure is an antibody production assisting method for assisting production of an antibody. The antibody production assisting method includes acquiring first time-series data indicating, in a time-series manner, an amount of metabolite generated as a result of culturing in a small scale a cell line for producing the antibody under a predetermined culture condition. The antibody production assisting method includes acquiring second time-series data indicating, in a time-series manner, an amount of metabolite generated as a result of culturing in a large scale the cell line under the predetermined culture condition. The antibody production assisting method includes specifying a difference between the first time-series data and the second time-series data. Also, the antibody production assisting method includes specifying a target metabolite derived from the difference. Additionally, the antibody production assisting method includes generating display data based on the target metabolite.

An antibody production assisting program according to a certain aspect of the present disclosure is a program for assisting production of an antibody. The antibody production assisting program causes a computer to execute acquiring of first time-series data indicating, in a time-series manner, an amount of metabolite generated as a result of culturing in a small scale a cell line for producing an antibody under a predetermined culture condition. The antibody production assisting program causes the computer to execute acquiring of second time-series data indicating, in a time-series manner, an amount of metabolite generated as a result of culturing in a large scale the cell line under the predetermined culture condition. The antibody production assisting program causes the computer to execute specifying of a difference between the first time-series data and the second time-series data. The antibody production assisting program causes the computer to execute specifying of a target metabolite derived from the difference. The antibody production assisting program causes the computer to execute generating of display data based on the target metabolite.

### ADVANTAGEOUS EFFECTS OF INVENTION

In the present disclosure, the display data based on the target metabolite derived from the difference between the first time-series data in the small scale and the second time-series data in the large scale is generated. Therefore, the manufacturer can recognize the target metabolite by visually recognizing an image based on the display data, and as a result, the manufacturer can presume a culture condition to be adjusted in the large scale. According to the present disclosure, it is thus possible to assist the production of the antibody in the large scale.

### BRIEF DESCRIPTION OF DRAWINGS

Fig. 1 is a configuration example of an assisting system according to a present embodiment.
Fig. 2 is a diagram illustrating a hardware configuration example of an information processing device.
Fig. 3 is a diagram illustrating an example of a graph illustrating, in a time-series manner, a culture parameter of a cell line A.
Fig. 4 is a diagram illustrating an example of a graph illustrating, in a time-series manner, a culture parameter of a cell line B.
Fig. 5 is a diagram illustrating an example of a graph illustrating, in a time-series manner, a plurality of metabolites generated for 10 days by culturing the cell lines.
Fig. 6 is a functional block diagram of an information processing device and the like.
Fig. 7 is a diagram illustrating an example of principal component score plots in principal component analysis.
Fig. 8 is a diagram illustrating an example of loading score plots in the principal component analysis.
Fig. 9 is a diagram illustrating an example of a first image.
Fig. 10 is a diagram illustrating an example of a metabolic map.
Fig. 11 is a diagram illustrating an example of a second image.
Fig. 12 is an enlarged view of images X of cell line A and cell line B.
Fig. 13 is a flowchart illustrating main processing of an information processing device 100.
Fig. 14 is a diagram illustrating an example of a third image.
Fig. 15 is a diagram illustrating an example of a fourth image.
Fig. 16 is a diagram illustrating an example of a fifth image.

### DESCRIPTION OF EMBODIMENTS

Hereinafter, an embodiment of the present disclosure will be described in detail with reference to the drawings. Note that the same reference signs will be applied to the same or corresponding parts in the drawings and description thereof will not be repeated.

### [Configuration example of assisting system]

In an embodiment, a manufacturer of an antibody (hereinafter, simply referred to as a "manufacturer" or a "user") cultures in a small scale a cell line under predetermined culture conditions. Also, the manufacturer produces an antibody by using the cultured cell line. The predetermined culture conditions are typically culture conditions that can be known by the manufacturer and is considered to be optimal. However, the culture conditions may be any culture conditions. Also, the manufacturer may specify the culture conditions that are considered to be optimal in the process of the small-scaled culture.

The manufacturer manufactures the antibody in the small scale under the predetermined culture conditions (the culture conditions that are considered to be optimal, for example) and also manufactures the antibody in a large scale to which scale-up of the manufacturing is applied in order to enhance efficiency of the manufacturing of the antibody. Theoretically, the antibody should be appropriately manufactured even in the large scale according to this method. However, there may be a case where it is not possible to appropriately manufacture the antibody in the large scale even if the antibody is manufactured under the culture conditions that are considered to be optimal in the large scale due to differences in environments and the like between the small scale and the large scale. Thus, production of antibodies used in antibody drug is assisted by an antibody production assisting method according to the present embodiment. More specifically, the antibody production assisting method allows the manufacturer to predict culture conditions to be set and values of the culture conditions in the large scale.

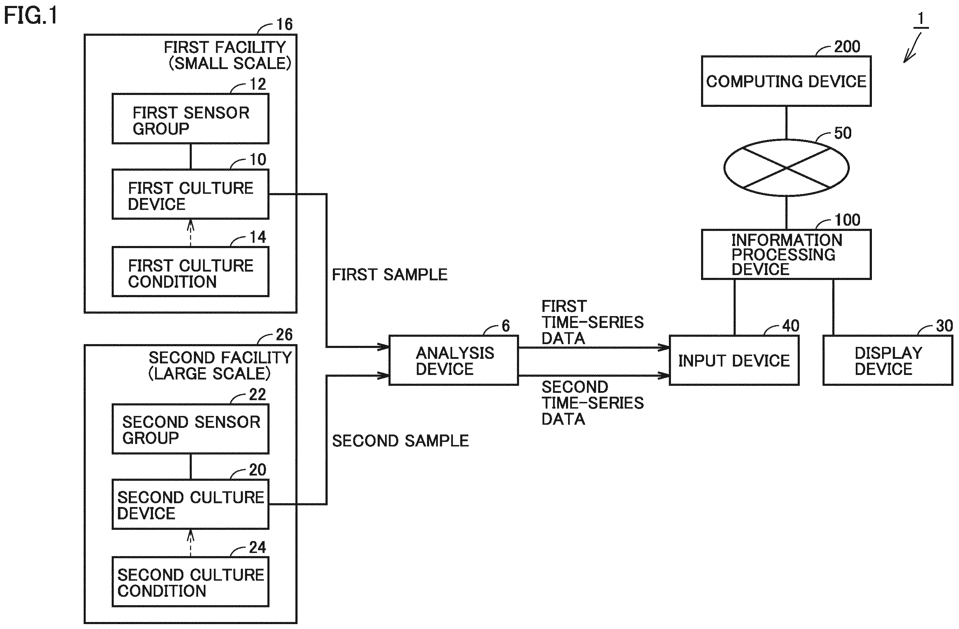

Fig. 1 is a configuration example of an assisting system 1 according to the present embodiment. Assisting system 1 includes a first culture device 10, a first sensor group 12, a second culture device 20, a second sensor group 22, an analysis device 6, an information processing device 100, a computing device 200, a display device 30, and an input device 40. Display device 30 and input device 40 are connected to information processing device 100.

First culture device 10 and first sensor group 12 are disposed in a first facility 16. First facility 16 is a facility where culture of cells is realized in a small scale. First culture device 10 cultures a cell line. The cell line is, for example, a CHO cell. Also, the scale of a culture tank of first culture device 10 is small and is, for example, a flask.

Second culture device 20 and second sensor group 22 are disposed in a second facility 26. Second facility 26 is a facility where culture of cells is realized in a large scale. In the present embodiment, second culture device 20 cultures the same cell line (that is, a CHO cell) as that of the first culture device 10. Also, antibodies produced by the cell lines in first culture device 10 and second culture device 20 are discharged from the cells to culture supernatants. Then, the antibodies are produced through purification from the culture supernatants. The cell line cultured by first culture device 10 will also be referred to as a "cell line A", and the cell line cultured by second culture device 20 will also be referred to as a "cell line B". In addition, both cell line A and cell line B are the aforementioned CHO cells. Moreover, cell line A and cell line B will also collectively be referred to as "cell lines".

The large scale means that the scale of manufacturing of the antibodies is larger than the small scale and is, for example, a scale of manufacturing in a plant or the like. Also, a manufacturing speed of the antibodies per unit time is higher in the large scale than in the small scale. In this manner, the large scale can realize mass production of the antibodies.

In addition, examples of the culture method include alternating tangential flow (ATF) culture as one kind of perfusion culture methods, fed-batch culture as one kind of feeding culture methods, and the like. Particularly, since a perfusion system is employed in the ATF culture, the manufacturer can realize continuous culture, that is, it is possible to culture the cell lines in a long-term culture period. In the present embodiment, the ATF culture method is employed for first culture device 10 and second culture device 20. Since the ATF culture method is employed, it is possible to culture the cells for a long period of time. In the present embodiment, it is assumed that the cell lines are cultured for a 10-day culture period.

In addition, a culture condition set for first culture device 10 will also be referred to as a "first culture condition 14", and a culture condition set for second culture device 20 will also be referred to as a "second culture condition 24". Hereinafter, first culture condition 14 and second culture condition 24 will also collectively be referred to as "culture conditions".

A culture condition that the manufacturer can set is, for example, at least one of an amount of basal medium, a composition of a basal medium, a flow rate of breeding, an amount of feed medium, a composition of a feed medium, a perfusion rate, a stirring speed, an amount of air blowing, an amount of carbon dioxide blowing, an amount of alkali addition, an amount of defoaming agent addition, and the like. The manufacturer sets at least one of these culture conditions as a culture condition through an operation or the like of a device corresponding to the culture condition. For the amount of carbon dioxide blowing, for example, the manufacturer sets the amount of carbon dioxide blowing by operating a carbon dioxide blowing device.

Also, each of first sensor group 12 and second sensor group 22 is configured of one or more sensors. Each of the one or more sensors detects a culture parameter corresponding to the sensor. The culture parameter includes a cell density, a cell viability, a cell diameter, a specific growth speed, an antibody production speed, pH, a temperature, dissolved oxygen (DO), an oxygen partial pressure, a carbon dioxide partial pressure, or the like. The specific growth speed is a proportion of an amount of increase in cells per unit time calculated from a difference from a previous (one day before, for example) cell density, or the like.

The manufacturer extracts, as a first sample, a part of a cell culture solution of cell line A cultured in first culture device 10. Also, the manufacturer extracts, as a second sample, a part of a cell culture solution of cell line B cultured in second culture device 20. Then, the manufacturer causes analysis device 6 to analyze the first sample and the second sample. At that time, analysis device 6 performs an operation for separating a culture solution supernatant and cells, a pre-analysis treatment is performed on each of them, and then analysis is performed. Analysis device 6 is, for example, a liquid chromatograph-mass spectrometry/mass spectrometry (LC-MS/MS). Also, the manufacturer also inputs the date and the time of the analysis to analysis device 6.

Analysis device 6 can measure the amount of each of metabolites (171 metabolites, for example) generated by culturing cell line A and cell line B. The manufacturer can select the number of metabolites to be measured and the types of metabolites. Then, analysis device 6 derives an analysis result (the amount of each of the plurality of metabolites) in an associated manner with the input date and time. The analysis results include a plurality of (for example, 171) items of time-series data indicating, in a time-series manner, each of amounts of the plurality of metabolites generated by culturing the cell lines. The time-series data may be data indicating directly the amount of each metabolite or may be data indicating indirectly the amount. In other words, the plurality of items of time-series data are data corresponding to each of amounts of the plurality of metabolites. In the present embodiment, the manufacturer causes analysis device 6 to analyze the samples once every day and causes it to derive analysis results. Note that the manufacturer may collectively preserve (for example, frozen preservation) the first sample and the second sample in the culture period (that is, 10 days). Then, the manufacturer may cause analysis device 6 to collectively analyze the first sample and the second sample in the culture period (that is, 10 days) at a predetermined timing.

Also, the manufacturer may cause a display device (not illustrated) of analysis device 6 to display the analysis result or may not cause the display device to display it every time the analysis result is derived. In this manner, the manufacturer causes analysis device 6 to derive the analysis results in the culture period (that is, 10 days). Hereinafter, the time-series data from first culture device 10 will be referred to as "first time-series data", and the time-series data from second culture device 20 will be referred to as "second time-series data".

Input device 40 is a device such as a keyboard or a mouse, for example, and receives a command from the manufacturer. Display device 30 is composed of a liquid crystal display (LCD) panel, for example, and displays information for the manufacturer. In a case where a touch panel is used as a user interface, input device 40 and display device 30 are integrally formed.

Information processing device 100 generates display data by executing predetermined processing on data transmitted from input device 40. Information processing device 100 may be implemented by a general-purpose computer or may be implemented by a computer dedicated for information processing device 100, for example. The general-purpose computer may be a personal computer (PC) or may be a tablet, for example. Note that at least a part of computing for generating the display data may be implemented as software by a CPU of information processing device 100 or may be implemented as hardware that performs processing independently from the CPU.

The manufacturer inputs the first time-series data and the second time-series data to input device 40. Information processing device 100 acquires the first time-series data and the second time-series data. Hereinafter, the first time-series data and the second time-series data will also collectively be referred to as "time-series data". The time-series data is data corresponding to the amounts of the plurality of metabolites in the culture period (10 days in the present embodiment).

Information processing device 100 and computing device 200 are configured to be able to communicate via a network 50. Once the time-series data is acquired from input device 40, then information processing device 100 outputs it to computing device 200. Computing device 200 executes predetermined computing, which will be described later, and outputs the computing result to information processing device 100. Information processing device 100 generates display data based on the computing result.

### [Hardware configuration of information processing device]

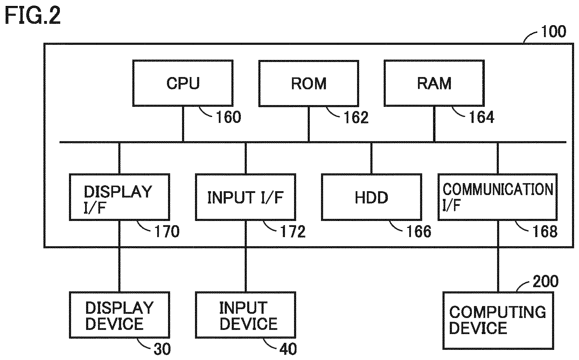

Fig. 2 is a diagram illustrating a hardware configuration example of information processing device 100. Referring to Fig. 2, information processing device 100 has, as main components, a central processing unit (CPU) 160, a read only memory (ROM) 162, a random access memory (RAM) 164, a hard disk drive (HDD) 166, a communication interface (I/F) 168, a display I/F 170, and an input I/F 172. The components are connected to each other via a data bus.

Communication I/F 168 is an interface for communicating with computing device 200. Display I/F 170 is an interface for communicating with display device 30. Input I/F 172 is an interface for communicating with input device 40.

ROM 162 stores a program to be executed by CPU 160. RAM 164 can temporarily store data generated by CPU 160 executing the program and data input via communication I/F 168. RAM 164 can function as a transitory data memory used as a work area. HDD 166 is a non-volatile storage device. A semiconductor storage device such as a flash memory may be employed instead of HDD 166.

The program stored in ROM 162 may be stored in a non-transitory recording medium and may be distributed as a program product. Alternatively, the program may be provided by an information provider as a product program that can be downloaded through what is called the Internet or the like. Information processing device 100 reads the program provided by the recording medium, the Internet, or the like. Information processing device 100 stores the read program in a predetermined storage region (in ROM 162, for example). CPU 160 can execute various kinds of processing by executing the program.

### [Concerning culture parameters]

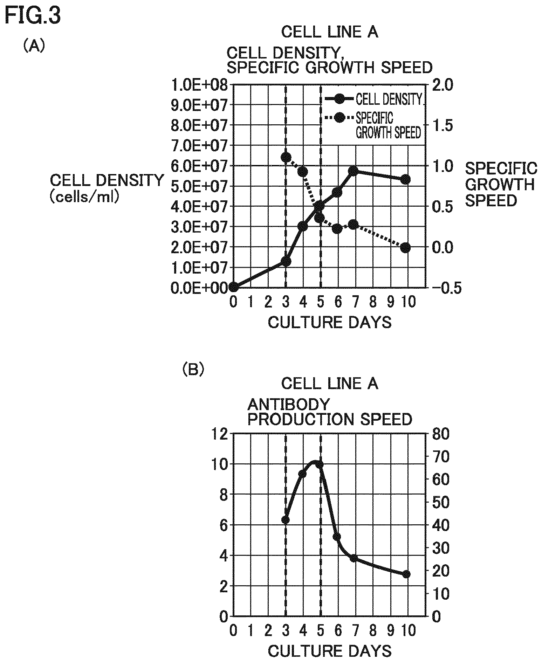

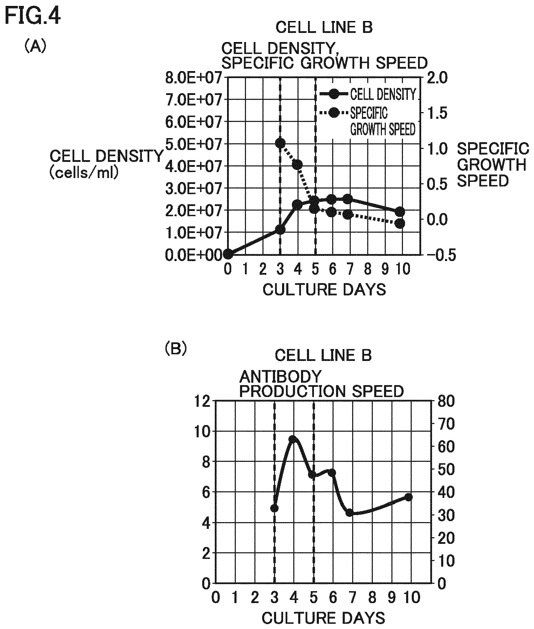

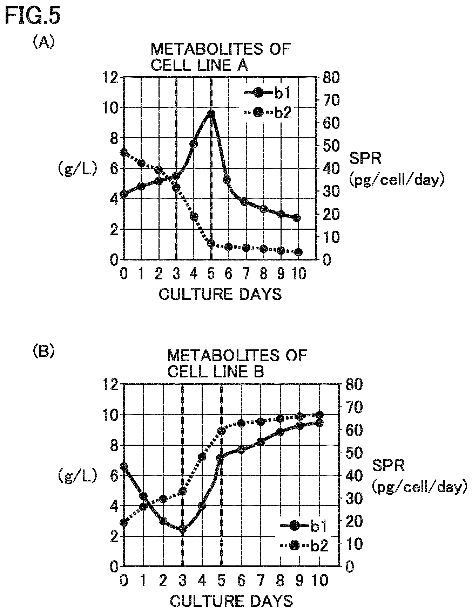

Next, culture parameters will be described. Fig. 3 is a graph illustrating, in a time-series manner, culture parameters of cell line A for 10 days. Fig. 4 is a graph illustrating, in a time-series manner, culture parameters of cell line B for 10 days. Fig. 5 is a graph illustrating, in a time-series manner, a plurality of metabolites generated by culturing the cell lines for 10 days. In the example of Fig. 5, a metabolite b 1 and a metabolite b2 are illustrated as the plurality of metabolites.

In Figs. 3 to 5, the horizontal axis represents the number of culture days. Also, the vertical axis in Fig. 3(A) represents a cell density and a specific growth speed as culture parameters of cell line A. The vertical axis in Fig. 3(B) represents an antibody production speed as a culture parameter of cell line A. The vertical axis in Fig. 4(A) represents a cell density and a specific growth speed as culture parameters of cell line B. The vertical axis in Fig. 4(B) represents an antibody production speed as a culture parameter of cell line B. The graphs in Figs. 3 to 5 are displayed on a predetermined display device (display device 30, for example), such that the manufacturer can visually recognize these graphs.

In the examples in Figs. 3 and 4, the cell densities, the specific growth speeds, and the antibody production speed of cell line A and cell line B significantly vary on the third to fifth days of the culture days as illustrated by the dashed lines. Moreover, in the example in Fig. 5, the amounts of metabolite b1 and metabolite b2 significantly vary on the third to fifth days of the culture days. Therefore, the manufacturer can presume occurrence of variations in metabolites that are highly related to variations in culture parameters on the third to fifth days of the culture days. Note that the period from the third day to the fifth day is typically a period from a cell growth period to a stationary period (the period during which convergence of cell growth and acceleration of antibody production occur).

In the process of manufacturing the antibodies in the large scale, the cultured cell lines are likely to be effected by changes in environment and the like inside the culture tank of second culture device 20, and variations in metabolites frequently occur. Thus, it is preferable that the manufacturer set (adjust) the culture condition in the large scale such that trends of variations in amounts of metabolites become equivalent between the small scale and the large scale. However, it is necessary for the manufacturer to set the culture condition in the large scale on the basis of limited monitoring results (the monitoring results in Figs. 3 to 5, for example). In the related art, manufacturers set culture conditions in a large scale on the basis of experience of the manufacturers themselves. Therefore, it may be difficult for some manufacturers to set appropriate culture conditions, and in this case, a problem that it is difficult to produce antibodies in the large scale may occur.

Incidentally, metabolites (such as metabolite b1 and metabolite b2 in Fig. 5) are typically produced in the process of manufacturing antibodies. Also, it is technically difficult to perform artificial control on the production of the metabolites. The inventor of the present disclosure has focused on a difference between the trend of variations in the amount of metabolite of cell line A cultured in the small scale and the trend of variations in the amount of metabolite of cell line B cultured in the large scale. Then, information processing device 100 specifies a target metabolite derived from the difference and displays an image corresponding to the target metabolite, which will be described later. Also, the target metabolite is a metabolite that is a principal factor of the above difference. Therefore, the manufacturer can presume the culture condition to be adjusted in the large scale on the basis of the image. Production of the antibodies in the large scale is assisted in the present embodiment.

### [Functional block diagram of information processing device]

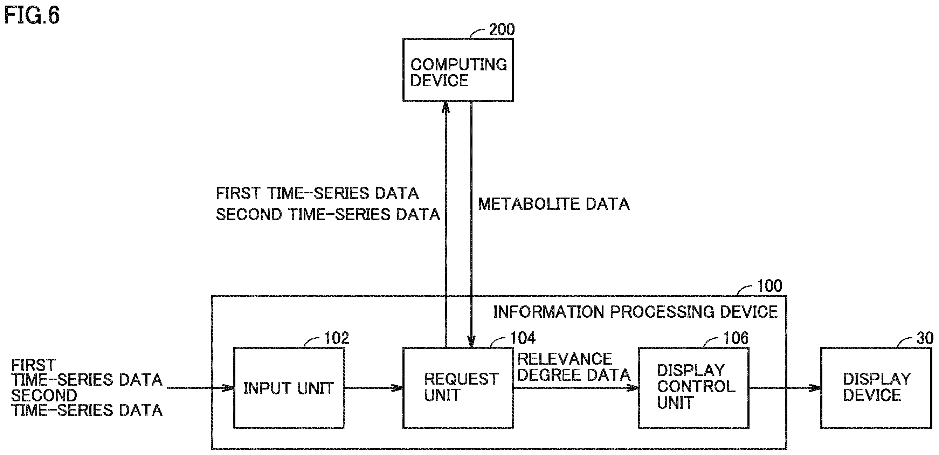

Fig. 6 is a functional block diagram of information processing device 100 and the like. Information processing device 100 has an input unit 102, a request unit 104, and a display control unit 106. The aforementioned first time-series data and second time-series data are input to input unit 102. Comma separated value (CSV) data, for example, is input as the first time-series data and the second time-series data to input unit 102. The manufacturer may manually input the first time-series data and the second time-series data to input unit 102. Also, analysis device 6 may input the first time-series data and the second time-series data to input unit 102. The first time-series data and the second time-series data are output to request unit 104. Request unit 104 outputs a request signal and the time-series data to computing device 200. The request signal is a signal for requesting metabolite data, which will be described later, from computing device 200.

Computing device 200 receives the request signal, the first time-series data, and the second time-series data. Computing device 200 executes principal component analysis on the first time-series data and the second time-series data. Computing device 200 generates metabolite data through the principal component analysis. The metabolite data is data indicating a target metabolite or the like derived from a difference between the first metabolite data and the second metabolite data. Computing device 200 transmits the metabolite data to information processing device 100.

Request unit 104 of information processing device 100 acquires the metabolite data. Request unit 104 transmits the metabolite data to display control unit 106. Display control unit 106 generates display data based on the metabolite data. Display control unit 106 causes display device 30 to display the image based on the display data.

### [Principal component analysis]

As described in Figs. 3 to 5, three culture parameters (cell densities, specific growth speeds, and antibody production speeds) of both cell line A and cell line B vary in the period from the third day to the fifth day in the culture period. Thus, samples of the three days (three samples) of each of cell line A and cell line B will be reviewed. The manufacturer inputs metabolite data (data indicating the amounts of 171 metabolites) of each of eighteen groups (= 2 × 3 × 3) to input device 40 (see Fig. 1). The metabolite data of the eighteen groups is transmitted to computing device 200, and computing device 200 executes multivariable analysis (for example, principal component analysis) on the metabolite data of the eighteen groups.

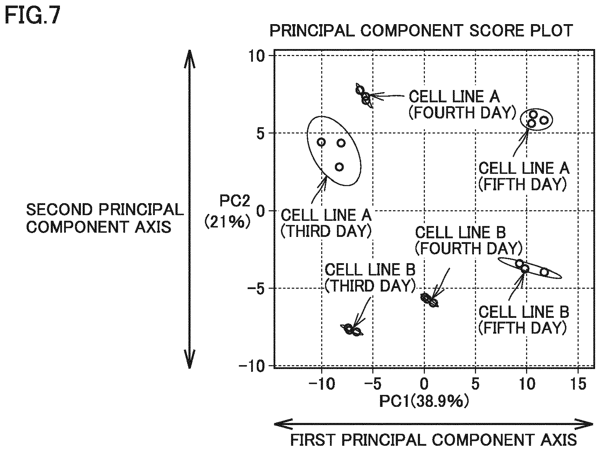

Fig. 7 is an example of principal component score plots by principal component analysis. The horizontal axis in Fig. 7 is a first principal component axis. Additionally, the vertical axis in Fig. 7 is a second principal component axis. The midpoint of the first principal component axis is "0", and the midpoint of the second principal component axis is "0".

In the example in Fig. 7, the three plot of cell line A (third day), the three plot of cell line A (fourth day), the three plot of cell line A (fifth day), the three plot of cell line B (third day), the three plot of cell line B (fourth day), and the three plot of cell line B (fifth day) are illustrated.

In the example in Fig. 7, the plots of cell line A and cell line B on the third day are distributed in a negative region of the first principal component axis. Also, the plots of cell line A and cell line B on the fifth day are distributed in a positive region of the first principal component axis. Therefore, the first principal component axis is an axis that can explain the time axis (the number of culture days).

Also, plots of cell line A are distributed in a positive region of the second principal component axis. Additionally, plots of cell line B are distributed in a negative region of the second principal component axis. Therefore, the second principal component axis is an axis that can explain the culture condition.

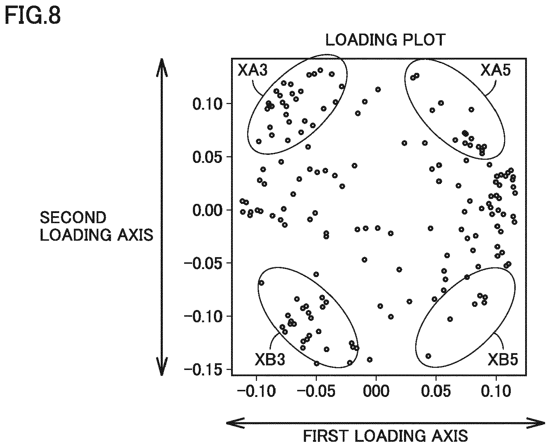

Fig. 8 is an example of loading score plots by principal component analysis. The plurality of plots illustrated in Fig. 8 are plots, each of which indicates each of the plurality of metabolites. In practice, a name of the metabolite is displayed in association with each of the plurality of plots in Fig. 8, the name of the metabolite is omitted in the example in Fig. 8.

In the example in Fig. 8, the horizontal axis is a first loading axis and corresponds to the first principal component axis in Fig. 8. Therefore, the first loading axis is an axis that can explain a time axis. Also, in the example of Fig. 8, the vertical axis is a second loading axis and corresponds to the second principal component axis in Fig. 8. Therefore, the second loading axis is an axis that can explain the culture condition.

In the example in Fig. 8, metabolite plots indicating large values (values whose absolute values are large) in a negative region of the first loading axis correspond to cell line A (third day) and are thus surrounded by a frame XA3. Also, metabolite plots indicating large values in a positive region of the first loading axis correspond to cell line A (fifth day) and are thus surrounded by a frame XA5. Metabolite plots indicating large values (values whose absolute values are large) in a negative region of the second loading axis correspond to cell line B (third day) and are thus surrounded by a frame XB3. Metabolite plots indicating large values in a positive region of the second loading axis correspond to cell line B (fifth day) and are thus surrounded by a frame XB5.

It is only necessary for the manufacture to view the names of the metabolites inside frame XA3 in Fig. 8 in a case where it is desired to recognize the metabolite of cell line A with a trend of a large value on the third day, for example. Also, the plurality of metabolite plots are present inside frame XA3. Information processing device 100 calculates the amount of PC1 principal component load and the amount of PC2 principal component load from a loading value (PC1 column) corresponding to a first principal component score and a loading value (PC2 column) corresponding to a second principal component score, for example. These values are correlation coefficients between the principal scores and each metabolite substance level, and it is thus possible to interpret an absolute value of the amount of principal component load of 0.4 or more as a component showing a slightly strong correlation and an absolute value of 0.7 or more as a component showing a strong correlation in accordance with a typical definition of the correlation coefficient. In this manner, a target metabolite that shows a strong correlation with a categorization pattern of a data group indicated by the principal component score plots is specified. The target metabolite is a metabolite derived from a difference between the amount of metabolite of cell line A cultured in a small scale and the amount of metabolite of cell line B cultured in a large scale. Also, specification of the target metabolite may be executed on the basis of a p value calculated by using the principal component loading amount and a significant level set by the manufacturer.

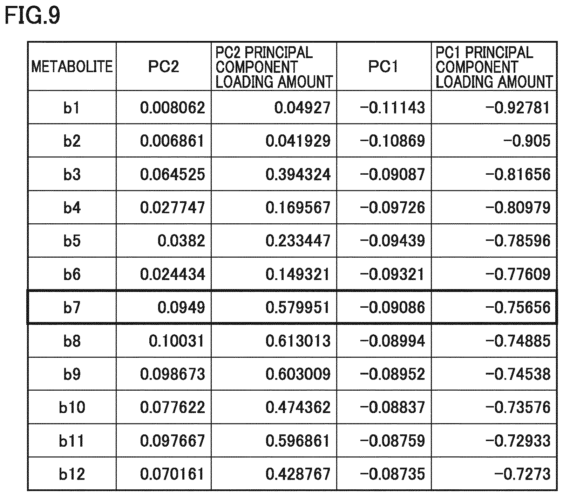

Fig. 9 is an example of an extraction result for the third day of cell line A. Information processing device 100 causes display device 30 to display an image of the extraction result. Information processing device 100 may cause display device 30 to display the image (first image) of the extraction result. The image of the extraction result corresponds to the "image based on the target metabolite" in the present disclosure. In the example in Fig. 9, plurality of metabolites b 1 to b 12 of plots surrounded by a frame XA3 are shown. Plurality of metabolites b1 to b 12 are candidate metabolites of the target metabolite. Furthermore, information processing device 100 specifies target metabolite b7 that is most attributable to the above difference from among candidate metabolites b 1 to b 12. Information processing device 100 displays target metabolite b7 in a different manner from the other metabolites (candidate metabolites). In the example in Fig. 9, information processing device 100 displays a cell corresponding to target metabolite b7 by surrounding it with a thick line.

Incidentally, the manufacturer can cause information processing device 100 to create a metabolic map. The metabolic map is a map that shows metabolites, metabolic pathways of the metabolites, and the like. Also, an application for assisting creation of the metabolic map has been downloaded on information processing device 100, and the manufacturer creates the metabolic map by using the application for assisting creation.

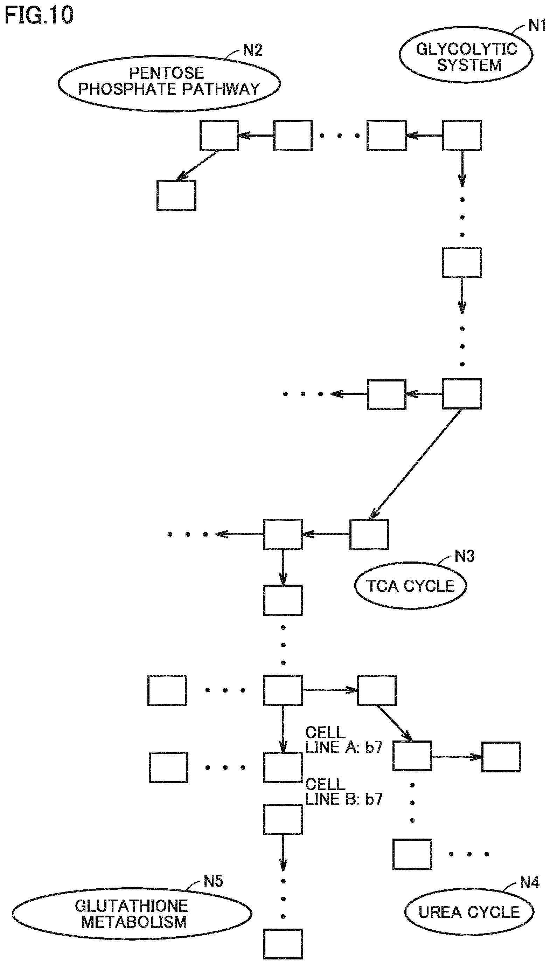

Fig. 10 is a diagram illustrating an example of the metabolic map. In the example in Fig. 10, an example of the metabolic map is omitted. In the example in Fig. 10, metabolites to be generated, metabolic pathways, and the like are shown. In the example in Fig. 10, identification information N1 for a metabolic pathway of a glycolytic system, identification information N2 for a metabolic pathway of a pentose phosphate pathway, identification information N3 for a metabolic pathway of a TCA cycle, identification information N4 for a metabolic pathway of a urea cycle, and identification information N5 for a metabolic pathway of a glutathione cycle are displayed as identification information for metabolic pathways.

In the example in Fig. 10, metabolite b7 of cell line A and metabolite b7 of cell line B are displayed. Also, names of the other metabolites are omitted.

Additionally, related information related to the metabolites is displayed below the names of the metabolites. The related information may include a graph or the like indicating a culture parameter (for example, a specific growth speed) of the metabolite in the culture period (10 days), for example. Also, the related information may include a graph indicating a time-series change in amount of metabolite. Moreover, the manufacturer may switch the related information (for example, the specific growth speed) to other related information (for example, cell viability) by executing a predetermined switching operation on input device 40.

Also, the manufacturer can estimate a culture condition to be adjusted in the large scale by visually recognizing the first image in Fig. 9 and the metabolic map in Fig. 10. For example, the manufacturer can estimate the culture condition to be adjusted by viewing each metabolite or the like included in the metabolic map around target metabolite b7 from the entire metabolic map. Furthermore, the manufacturer can also estimate how much the culture condition to be adjusted is to be adjusted by viewing the metabolic map in the surroundings.

Next, another display image that display device 30 displays will be described. For example, if the manufacturer designate a cell of a name of a metabolite from among names of a plurality of metabolites b1 to b12 (including target metabolite b7) displayed in the first image in Fig. 9, for example, an image (second image) of only a metabolic map in the surroundings of the metabolite may be displayed, and a metabolic map at other locations may not be displayed.

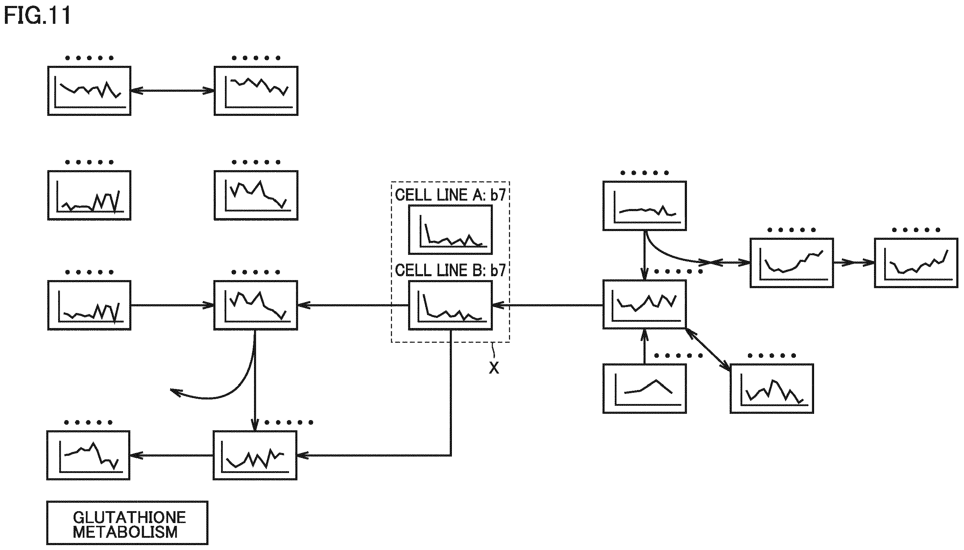

Fig. 11 is an example of an image (second image) of the metabolic map around the designated metabolite. The example in Fig. 11 is the metabolic map displayed in a case where target metabolite b7 is designated from among the plurality of metabolites in Fig. 9. In this example, target metabolite b7 is a glutathione metabolic pathway, and a metabolic map corresponding to the glutathione metabolic pathway is thus displayed in the metabolic map. In this manner, only the metabolic map around the designated metabolite is displayed from among the plurality of metabolites that are derived from the above differences, and the metabolic map at another location is not displayed in the example in Fig. 11. Furthermore, related information of target metabolite b7 is displayed for both cell line A and cell line B in the example in Fig. 11. The related information is a graph illustrating a culture parameter (for example, a specific growth speed) of the metabolite in the culture period (10 days), for example. Also, the manufacturer may switch the related information (specific growth speed) to other related information (cell viability) by executing a predetermined switching operation on input device 40.

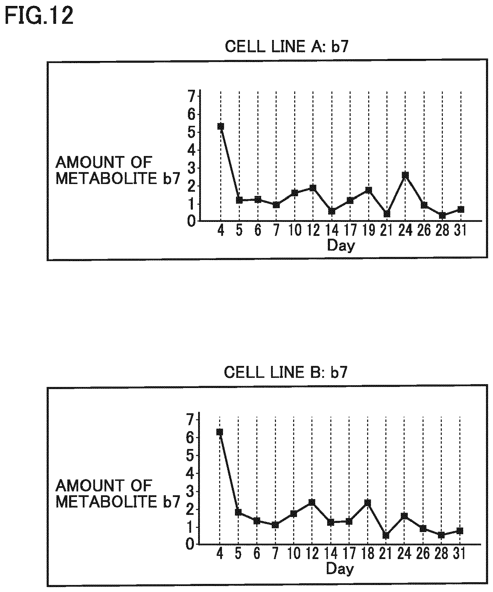

Fig. 12 is an enlarged view of an image X of cell line A and cell line B in Fig. 11. Note that information processing device 100 can cause the screen in Fig. 12 to be displayed by the manufacturer performing a predetermined enlarging operation on the screen in Fig. 11. In the example of Fig. 12, a time-series change in each of the amount of metabolite b7 of cell line A and the amount of metabolite b7 of cell line B is displayed. Also, the manufacturer may be able to cause other information (for example, a culture parameter) to be displayed as displayed information by performing a predetermined operation. With such a configuration, the manufacturer can estimate the culture condition to be adjusted in the large scale on the basis of the amounts of the time-series changes in the target metabolite of cell line A and the target metabolite of cell line B, the culture parameter, and the other locations in the metabolic map.

### [Flowchart of information processing device]

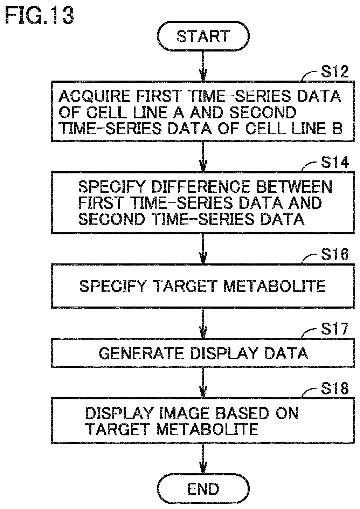

Fig. 13 is a flowchart illustrating main processing of information processing device 100. In the example of Fig. 13, information processing device 100 acquires the first time-series data and the second time-series data first in Step S12. Next, information processing device 100 outputs a request signal, the first time-series data, and the second time-series data to computing device 200 in Step S14. Computing device 200 executes principal component analysis on the first time-series data and the second time-series data. Computing device 200 generates the metabolite data by the principal component analysis. Computing device 200 transmits the metabolite data to information processing device 100, and information processing device 100 thereby acquires the metabolite data. Then, information processing device 100 specifies a difference between the first time-series data and the second time-series data on the basis of the metabolite data (see Fig. 7 and Fig. 8). Next, information processing device 100 specifies target metabolite b7 in Step S16 (see Fig. 9). Next, information processing device 100 generates display data based on target metabolite b7 in Step S17. Then, information processing device 100 displays an image (the above first image or second image) based on target metabolite b7 on display device 30 in Step S18.

### [Other display images]

Next, other display images of images to be displayed by display device 30 will be described. For example, the configuration in which the image of only the metabolic map around target metabolite b7 that has been designated in the first image in Fig. 9 is displayed has been described in Figs. 11 and 12.

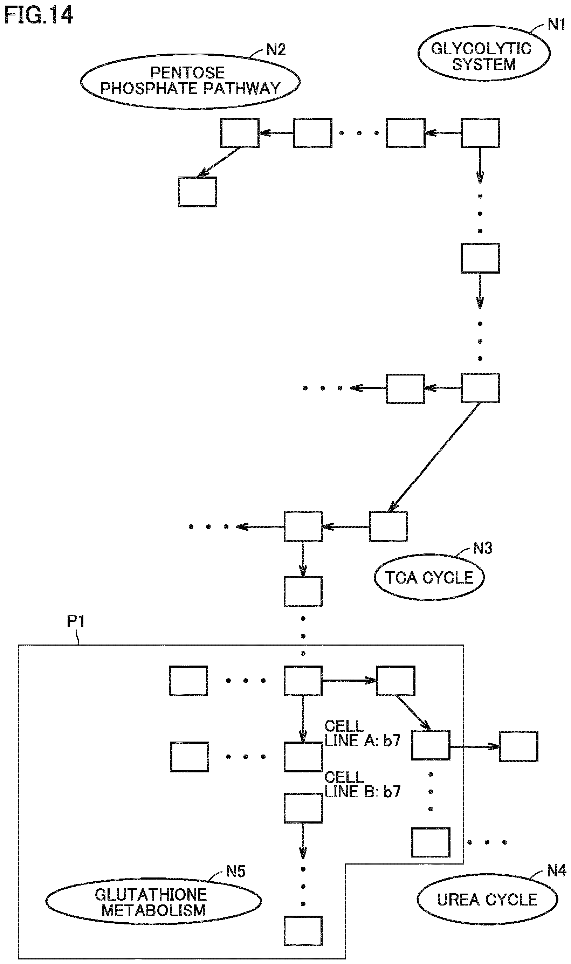

However, information processing device 100 may display the metabolic map displayed in a case where designation has been made from among the plurality of metabolites as an image (third image) in a different manner from the metabolic map at the other location. Fig. 14 is a diagram illustrating an example of the third image. In the example in Fig. 14, a glutathione metabolic pathway of target metabolite b7 is displayed by being surrounded by a frame P1. In other words, the metabolic map of the glutathione metabolic pathway is displayed in a different manner from the metabolic map at another location. With such a configuration, the manufacturer can more clearly identify the metabolic map around the designated metabolite (target metabolite). Therefore, the manufacturer can more clearly presume the culture condition to be adjusted on the basis of the metabolic map around the designated metabolite. Note that the different manner is not limited to that in the example in Fig. 14 and another manner may be used. For example, blinking or the like of the metabolic map to be displayed in a case where designation has been made from among the plurality of metabolites may be performed.

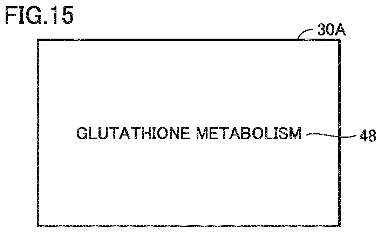

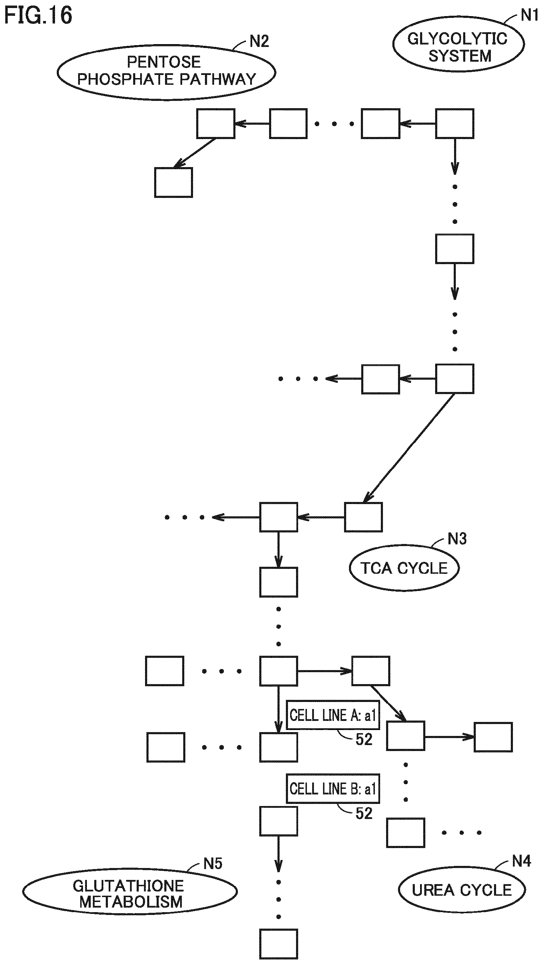

Also, information processing device 100 may display a fourth image including a name of a metabolic pathway of the designated metabolite. Fig. 15 is an example of the fourth image. As illustrated in Fig. 15, a name image 48 of the metabolic pathway is displayed in a display region 30A on display device 30. In the example of Fig. 15, name image 48 is a character image of "Glutathione metabolism". With such a configuration, the manufacturer can recognize the metabolic pathway of the target metabolite b7 (the metabolite designated by the manufacturer). Therefore, the manufacturer can more clearly presume the culture condition to be adjusted on the basis of the metabolic pathway. Note that display region 30A is not described in Fig. 14 and Fig. 16, which will be described later.

Also, information processing device 100 may display the fourth image for enabling the designated metabolite to be identifiable in the metabolic map. Fig. 16 is an example of the fifth image. As illustrated in Fig. 16, information processing device 100 displays the metabolic map. Along with this, information processing device 100 displays the designated metabolite in a different manner from the other metabolites in the metabolic map. In the example in Fig. 16, it is assumed that target metabolite b7 has been designated by the manufacturer. In this case, information processing device 100 displays designated target metabolite b7 in a different manner from the other metabolites. In the example in Fig. 15, the name of target metabolite b7 is displayed in a manner in which it is surrounded by frame 52. With this configuration, the manufacturer can recognize the metabolite derived from the aforementioned difference. Therefore, the manufacturer can more clearly presume the culture condition to be adjusted in the large scale on the basis of the metabolite.

Also, the case where there are multiple (twelve in the example in Fig. 9) metabolites that are derived from the aforementioned difference has been described in the present embodiment. However, in a case where there is one metabolite that is derived from the aforementioned difference, any image out of the third image and the fifth image may be displayed for the one metabolite. Also, display of any of the aforementioned first image to the fifth image may be switched by an operation of the manufacturer.

### [Modification examples]

(1) In the aforementioned embodiment, the configuration in which information processing device 100 generates the display data and causes display device 30 to display the image based on the display data has been described. However, a configuration in which information processing device 100 does not cause display device 30 to display the image may be employed. In a case of such a configuration, the manufacturer causes a recording medium that can record information thereon to be connected to information processing device 100, for example. Then, information processing device 100 causes the recording medium to store the display data. The manufacturer may connect the recording medium storing the display data thereon to a different display device and cause the different display device to display the image based on the display data. Even with such a configuration, effects that are similar to those of the aforementioned embodiment are achieved.
(2) In the aforementioned embodiment, the configuration in which analysis device 6 and information processing device 100 are separate devices has been described. However, analysis device 6 may execute at least a part of processing executed by information processing device 100. For example, analysis device 6 may display the image that display device 30 is caused to display as described above.
(3) In the aforementioned embodiment, the example in which single information processing device 100 executes all the processes has been described. However, one or multiple processes may be executed by another information processing device. The plurality of information processing device may be disposed at remote locations from each other. For example, a certain computer may execute the acquisition of the degrees of relevance, and another computer disposed at a remote location may execute the generation of the display data. A plurality of other computers may be present and perform generation of mutually different display data.

### [Aspects]

Those skilled in the art will understand that the aforementioned plurality of illustrative embodiments are specific examples of the following aspects.

(Clause 1) An antibody production assisting method according to an aspect is an assisting method for assisting production of an antibody including: acquiring first time-series data indicating, in a time-series manner, an amount of metabolite generated as a result of culturing in a small scale a cell line for producing the antibody under a predetermined culture condition; acquiring second time-series data indicating, in a time-series data, an amount of metabolite generated as a result of culturing in a large scale the cell line under the predetermined culture condition; specifying a difference between the first time-series data and the second time-series data; specifying a target metabolite derived from the difference; and generating display data based on the target metabolite.

With such a configuration, the display data based on the target metabolite derived from the difference between the first time-series data in the small scale and the second time-series data in the large scale is generated. Therefore, the manufacturer can recognize the target metabolite by visually recognizing an image based on the display data, and as a result, the manufacturer can presume a culture condition to be adjusted in the large scale and assist production of the antibody in the large scale.

(Clause 2) In the antibody production assisting method described in the first clause, the antibody production assisting method further includes: causing a display device to display an image based on the display data.

With such a configuration, an image related to the display data based on the target metabolite is displayed. Therefore, the manufacturer can presume a culture condition to be adjusted in the large scale by visually recognizing the image.

(Clause 3) In the antibody production assisting method described in the second clause, the causing a display device to display includes causing a display device to display, in an associated manner, a time-series change in amount of the target metabolite in the small scale and a time-series change in amount of the target metabolite in the large scale.

With such a configuration, it is possible to facilitate manufacturer's visual recognition of culture parameters in the small scale in regard to the target metabolite and culture parameters in the small scale in regard to the target metabolite.

(Clause 4) In the antibody production assisting method described in the second clause or the third clause, the causing a display device to display includes causing a display device to display a metabolic map in a location surrounding the target metabolite in a manner different from a metabolic map at another location.

With such a configuration, the manufacturer can recognize the metabolic map in the location surrounding the target metabolite. Therefore, the manufacturer can more clearly presume a culture condition to be adjusted in the large scale on the basis of the metabolic map.

(Clause 5) In the antibody production assisting method described in the second clause or the third clause, the causing a display device to display includes causing a display device to display a metabolic map in a location surrounding the target metabolite and not displaying a metabolic map at another location.

With such a configuration, the manufacturer can recognize the metabolic map in the location surrounding the target metabolite. Therefore, the manufacturer can more clearly presume a culture condition to be adjusted in the large scale on the basis of the metabolic map.

(Clause 6) In the antibody production assisting method according to any one of the second clause to the fifth clause, the causing a display device to display includes causing a display device to display a name of a metabolic pathway of the target metabolite.

With such a configuration, the user can recognize the metabolic pathway of the target metabolite. Therefore, the user can more clearly presume a culture condition to be adjusted in the large scale on the basis of the metabolic pathway.

(Clause 7) In the antibody production assisting method according to any one of the second clause to the sixth clause, the causing a display device to display includes causing a display device to display the target metabolite in an identifiable manner in a metabolic map.

With such a configuration, the user can recognize the target metabolite. Therefore, the user can more clearly presume a culture condition to be adjusted in the large scale on the basis of the target metabolite.

(Clause 8) In the antibody production assisting method according to any one of the second clause to the seventh clause, the specifying the difference includes specifying the difference by using principal component analysis.

With such a configuration, it is possible to specify the difference on the basis of the principal component analysis which is an existing technique.

(Clause 9) An antibody production assisting program according to an aspect is a program for assisting production of an antibody, the antibody production assisting program causes a computer to execute: acquiring of first time-series data indicating, in a time-series manner, an amount of metabolite generated as a result of culturing in a small scale a cell line for producing the antibody under a predetermined culture condition; acquiring of second time-series data indicating, in a time-series manner, an amount of metabolite generated as a result of culturing in a large scale the cell line under the predetermined culture condition; specifying of a difference between the first time-series data and the second time-series data; specifying of a target metabolite derived from the difference; and generating of display data based on the target metabolite.

With such a configuration, the display data based on the target metabolite derived from the difference between the first time-series data in the small scale and the second time-series data in the large scale is generated. Therefore, the manufacturer can recognize the target metabolite by visually recognizing an image based on the display data, and as a result, the manufacturer can presume a culture condition to be adjusted in the large scale and assist production of the antibody in the large scale.

The embodiments disclosed herein should be considered as being illustrative examples and not limitations in any sense. The scope of the present disclosure is indicated by the scope of the claims rather than the above description of the embodiments, and is intended to include all modifications within meanings and a scope equivalent to the scope of the claims.

### REFERENCE SIGNS LIST

1 Assisting system; 6 Analysis device; 10 First culture device; 12 First sensor group; 14 First culture condition; 16 First facility; 20 Second culture device; 22 Second sensor group; 24 Second culture condition; 26 Second facility; 30 Display device; 30A Display region; 40 Input device; 48 Name image; 50 Network; 100 Information processing device; 102 Input unit; 104 Request unit; 106 Display control unit; 162 ROM; 164 RAM; 200 Computing device

## Claims

1. An antibody production assisting method for assisting production of an antibody, the method comprising:
acquiring first time-series data corresponding to an amount of metabolite generated as a result of culturing in a small scale a cell line for producing the antibody under a predetermined culture condition;
acquiring second time-series data corresponding to an amount of metabolite generated as a result of culturing in a large scale the cell line under the predetermined culture condition;
specifying a difference between the first time-series data and the second time-series data;
specifying a target metabolite derived from the difference; and
generating display data based on the target metabolite

2. The antibody production assisting method according to claim 1, further comprising: causing a display device to display an image based on the display data.

3. The antibody production assisting method according to claim 2, wherein the causing a display device to display includes causing a display device to display ,in an associated manner, a time-series change in amount of the target metabolite in the small scale and a time-series change in amount of the target metabolite in the large scale.

4. The antibody production assisting method according to claim 2, wherein the causing a display device to display includes causing a display device to display a metabolic map in a location surrounding the target metabolite in a manner different from a metabolic map at another location.

5. The antibody production assisting method according to claim 2, wherein the causing a display device to display includes causing a display device to display a metabolic map in a location surrounding the target metabolite and not displaying a metabolic map at another location.

6. The antibody production assisting method according to claim 2, wherein the causing a display device to display includes causing a display device to display a name of a metabolic pathway of the target metabolite.

7. The antibody production assisting method according to claim 2, wherein the causing a display device to display includes causing a display device to display the target metabolite in an identifiable manner in a metabolic map.

8. The antibody production assisting method according to claim 1, wherein the specifying the difference includes specifying the difference by using principal component analysis.

9. An antibody production assisting program for assisting production of an antibody,
the program causing a computer to execute:
acquiring of first time-series data indicating, in a time-series manner, an amount of metabolite generated as a result of culturing in a small scale a cell line for producing the antibody under a predetermined culture condition;
acquiring of second time-series data indicating, in a time-series manner, an amount of metabolite generated as a result of culturing in a large scale the cell line under the predetermined culture condition;
specifying of a difference between the first time-series data and the second time-series data;
specifying of a target metabolite derived from the difference; and
generating of display data based on the target metabolite.
